# FASCICULE DE BREVET EUROPEEN

(11) **EP 0 519 922 B1**
(45) Date de publication et mention de la délivrance du brevet: **12.10.1994**
(21) Numéro de dépôt: 90912443.0
(22) Date de dépôt: 27.07.1990
(51) Int. Cl.: A61M 5/32

(54) **SERINGE A AIGUILLE AUTO-ESCAMOTABLE**
SPRITZE MIT SICH AUTOMATISCH ZURÜCKZIEHENDER NADEL
SYRINGE WITH SELF-RETRACTING NEEDLE

(30) Priorité: 08.03.1990 FR 9002964
(43) Date de publication de la demande: 30.12.1992
(73) Titulaire: BLUE STAR CORPORATION S.A., Montevideo (UY)
(72) Inventeur: Guerineau, Jean, Issy Les Moulineaux 92130 (FR); Poirier, Dominique, Issy les Moulineaux 92130 (FR)
(74) Mandataire: Bertrand, Didier
(86) Numéro de dépôt international: FR9000572
(87) Numéro de publication internationale: WO9113643

(56) Documents cités:
- EP-A- 0 288 003
- EP-A- 0 307 367
- EP-A- 0 405 039

## Description

La présente invention concerne les seringues médicales, c'est-à-dire les appareils servant à injecter à l'aide d'une aiguille creuse une substance, généralement médicamenteuse, dans le corps du patient, par voie intramusculaire, sous-cutanée ou intraveineuse. Cette opération est effectuée, soit par un personnel médical, soit par le patient lui-même, on parle alors d'auto-injection.

L'emploi s'est maintenant généralisé des seringues dites à usage unique, vendues stériles dans un emballage clos et ne devant pas être réutilisées, ceci afin d'éviter tout risque de contamination accidentelle d'un patient à l'autre. Mais ce matériel, largement répandu, ne protège pas le personnel médical d'une piqûre accidentelle et n'interdit pas matériellement le réemploi de la seringue si l'usager ne procède pas à sa destruction volontaire après emploi.

Le document EP-A-0 307 367 décrit une seringue à aiguille auto-escamotable, comprenant un corps portant à une extrémité des moyens de support d'une aiguille d'injection, un piston mobile dans ledit corps, un fourreau protecteur à l'intérieur duquel est susceptible de coulisser ledit corps, et un ressort disposé entre le fourreau et le corps. Il est aussi prévu des moyens d'accrochage du fourreau sur le corps comprenant un premier et un second épaulements sur la paroi externe du corps et au moins un premier ergot souple porté par le fourreau de manière à coopérer en butée avec le premier épaulement pour définir une première position du corps par rapport au fourreau de blocage dans un premier sens, le ressort étant comprimé et l'aiguille sortant du fourreau dans cette première position, et avec le second épaulement pour définir une seconde position de blocage dans le même sens, le ressort étant au moins partiellement débandé et l'aiguille étant rentrée dans le fourreau dans cette seconde position. Le passage de la première position à la seconde position se fait par la coopération du piston avec ledit premier ergot souple pour libérer en fin de course dudit piston l'ergot du premier épaulement et provoquer ainsi sous l'effet du ressort le recul du corps dans le fourreau jusqu'à son blocage en seconde position.

Une telle construction donne partiellement satisfaction en ce sens que, de fait, après injection, l'aiguille se trouve bien rétractée dans son fourreau. En revanche, la fabrication de cette seringue est assez compliquée, ce qui en grève le prix, et elle est par ailleurs peu fiable. En effet, la coopération du piston avec les ergots de blocage n'est pas directe, mais se fait par l'intermédiaire d'une pièce supplémentaire constituée par une bague élastique, que des personnes peu scrupuleuses peuvent déformer et rendre utilisable.

D'autre part, selon le document cité, on empêche l'aiguille de ressortir en prévoyant un orifice du fourreau très réduit, juste suffisant pour le passage de l'aiguille, laquelle est pré-orientée de façon oblique de manière qu'après sa rétraction dans le fourreau, elle ne puisse plus ressortir par ledit orifice. Une telle disposition présente l'inconvénient qu'il est impossible de prévoir une seringue à aiguilles interchangeables puisque les embouts de ces aiguilles ne pourraient pas passer par l'orifice du fourreau. Or, il est souvent pratique pour le personnel médical de pouvoir adapter sur la seringue une aiguille à sa guise, par exemple une aiguille de gros diamètre pour aspirer le liquide dans la seringue, et une aiguille de faible diamètre pour l'injection proprement dite.

L'invention vise à proposer une nouvelle seringue dépourvue de cet inconvénient.

La seringue conforme à l'invention est du type général, connu par le document précité et rappelé plus haut. Elle s'en distingue en ce qu'elle comprend au moins un second ergot souple et un troisième épaulement, de sens contraire respectivement au premier ergot souple et aux deux premiers épaulements, positionnés respectivement sur le fourreau et la paroi du corps pour bloquer le corps dans le fourreau, lorsqu'il est dans la seconde position de blocage, dans le sens inverse du premier sens.

Ainsi grâce à ce second ergot et à ce troisième épaulement, le blocage du corps dans le fourreau en vue d'empêcher l'aiguille de ressortir, ne nécessite plus de limiter l'orifice de sortie du fourreau. Aussi est-il possible et souhaitable, selon l'invention, que la partie inférieure du corps soit susceptible de recevoir un embout porte-aiguille et que l'extrémité inférieure du fourreau comporte un orifice de passage pour ledit embout.

Avantageusement, au moins certains des ergots et de préférence tous sont souples et réalisés d'un seul tenant avec le fourreau par exemple dans une matière plastique telle que du polypropylène, du polyéthylène ou du polystyrène. Naturellement, le fourreau lui-même peut être réalisé en une seule partie ou plusieurs parties assemblées si cela est plus simple.

Très avantageusement, au moins certains des ergots, et de préférence tous, sont protégés de l'extérieur par une paroi en forme de manchon solidaire du fourreau, cette paroi pouvant être rapportée ou bien ne former qu'un avec le fourreau. Dans un mode de réalisation, cette paroi est constituée par le fourreau lui-même (ou une partie du fourreau), les ergots étant formés à l'intérieur dudit fourreau.

Selon un premier mode de réalisation, les ergots sont constitués de simples découpes, avec épaississement éventuel, de la paroi du fourreau, situées en vis-à-vis dans des lumières formées sur cette paroi. Les épaulements du corps sont, eux, avantageusement formés par les faces frontales de bagues ou filets sensiblement carrés disposés par moulage à un endroit approprié du corps cylindrique.

Selon un second mode de réalisation, le corps comprend un mandrin porte-aiguille et au moins certains des ergots, notamment les seconds, c'est-à-dire les ergots "anti-retour" sont prévus à l'intérieur du fourreau, à son extrémité, pour pouvoir entourer ledit mandrin dans la première position, et pour le laisser échapper vers la seconde position et l'y bloquer. Avantageusement, le mandrin porte-aiguille comporte une zone amincie, de plus faible résistance, pouvant casser sous une pression exagérée.

Selon un troisième mode de réalisation, les premiers et seconds ergots sont réalisés à l'intérieur d'un manchon rapporté constituant l'extrémité proximale du fourreau.

Il est avantageux d'engager une extrémité du ressort dans une zone de moindre résistance ménagée sur le corps, à l'endroit du rattachement de l'aiguille ou de l'embout porte-aiguille, qui permet à l'aiguille de se détacher du corps en cas de tentative de réutilisation.

Le piston comporte une partie susceptible de venir directement en contact avec les premiers ergots de blocage, en fin de course d'injection : cette partie peut être réalisée sous forme d'un élargissement de la tige de piston, ou d'une jupe portée par le piston.

D'autres caractéristiques et avantages de l'invention apparaîtront à la lecture de la description suivante. Il sera fait référence aux dessins annexés sur lesquels :
- la figure 1 est une vue en coupe d'une seringue conforme à un premier mode de réalisation de l'invention, selon la ligne I-I de la figure 2, en position armée ;
- la figure 2 est une vue de côté de la même seringue ;
- la figure 3 est une vue en coupe de la seringue de la figure 1 après rétraction du corps dans le fourreau;
- la figure 4 est une vue partielle en coupe de l'extrémité distale d'un second mode de réalisation de seringue, en position armée ;
- la figure 5 est une vue analogue à celle de la figure 4, en position désarmée ;
- la figure 6 est une vue analogue à celle de la figure 5, avec une variante du mode de fixation de l'aiguille ;
- la figure 7 est une vue partielle du corps de l'extrémité proximale d'un troisième mode de réalisation de seringue, selon la ligne VII-VII de la figure 8 ;
- la figure 8 est une vue de dessus du même mode de réalisation ;
- la figure 9 et une vue en coupe selon la ligne IX-IX de la figure 8.

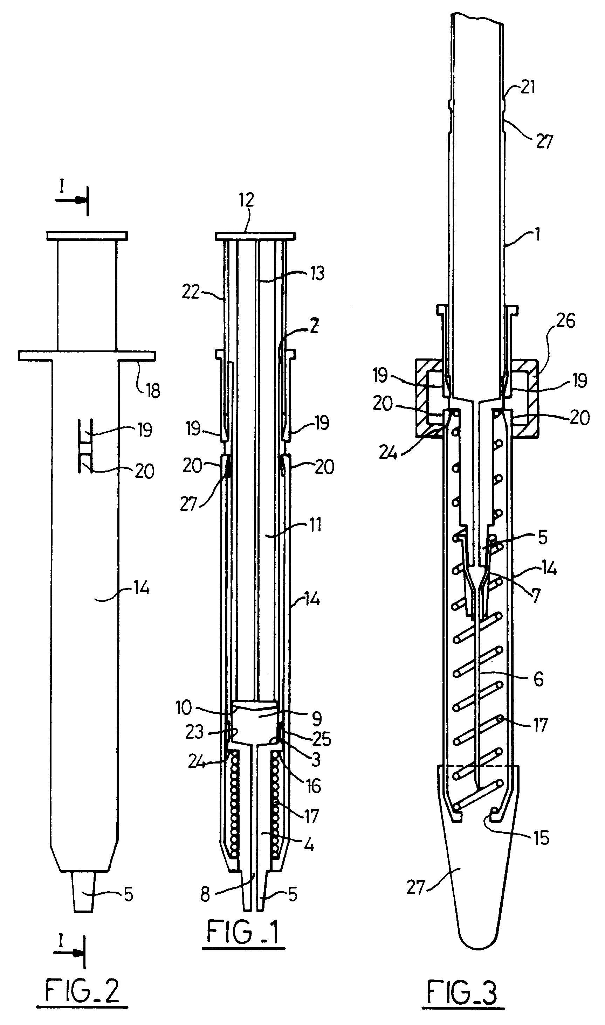

Les figures 1 à 3 montrent un ensemble de seringue constitué d'un corps 1 sensiblement cylindrique et comportant une extrémité ouverte et une extrémité partiellement fermée ou fond 3.

Tenant au fond 3 est formé un embout 4, 5 comportant une partie cylindrique 4 suivie d'une partie tronconique 5 servant de téton de fixation d'une aiguille classique 6 à embout de fixation tronconique 7. L'embout 4, 5 est percé d'un alésage 8 faisant communiquer l'intérieur 9 du corps avec l'alésage de l'aiguille 6.

Par l'extrémité ouverte 2 du corps 1, peut pénétrer dans le corps 1 un piston 13 à étanchéité 10, à tige cruciforme 11 et poussoir 12, coulissant librement à l'intérieur du corps 1. Une jupe périphérique 22 liée au poussoir 22 peut recouvrir en partie le corps 1.

Un fourreau 14 sensiblement cylindrique est monté coulissant sur le corps 1. A une de ses extrémités, sa paroi se rétrécit pour former un orifice 15 suffisant pour le passage du téton 5 de fixation de l'aiguille, et dont le contour peut servir d'appui à un ressort de compression 17 dont l'autre extrémité s'appuie sur un épaulement 16 du corps 1, au niveau de l'extrémité 3. Le ressort 17 est maintenu pendant sa compression autour de la partie 4 de l'embout 4, 5 du corps.

Le fourreau 14 comporte à son autre extrémité une collerette 18 d'appui des doigts.

Dans la paroi du fourreau 14 sont pratiquées deux lumières rectangulaires latérales en partie formées par deux languettes ou ergots élastiquement flexibles et de sens opposé 19 et 20.

Les premiers ergots 19 coopèrent avec un premier épaulement périphérique 21 formé sur la paroi externe du corps 1 pour effectuer un premier blocage du corps 1 dans le fourreau 14, dans la position des figures 1 ou 2 correspondant à l'armement de la seringue , où le ressort 17 est comprimé. Les mêmes ergots 19 sont susceptibles de s'écarter sous l'action de la jupe 22 quand le piston 13 est enfoncé dans le corps 1. Le ressort 17 se détend alors et écarte le fourreau 14 du corps 1 jusqu'à la seconde position de blocage dans laquelle les ergots 19 coopèrent avec un second épaulement périphérique 23 (position montrée sur la figure 3) situé à la base du corps 1.

Les ergots 19 et l'épaulement 23 empêchent donc de retirer le corps 1 et son aiguille 6 du fourreau 14 qui la recouvre alors.

Les seconds ergots 20, agissant au sens opposé des ergots 19, sont destinés à interdire une nouvelle sortie de l'aiguille 6 hors du fourreau, sortie que la largeur de l'orifice 15 ne permet pas d'empêcher à elle seule. Pour cela, les ergots 20 coopèrent avec un troisième épaulement 24 (de sens contraire aux épaulements 21 et 23), par exemple constitué par le bord de l'épaulement 16 d'appui du ressort 17. De la sorte, après l'injection, le corps 1 reste bloqué, aussi bien en avant qu'en arrière, dans la position représentée figure 3 et l'aiguille 6 est inaccessible. Naturellement, le piston 13, tout à fait classique, peut être retiré du corps 1 (il n'a d'ailleurs pas été représenté en figure 3).

Les ergots 19 et 20 sont de préférence réalisés sous forme de languettes flexibles ayant une certaine sur-épaisseur formant vers l'intérieur du fourreau une saillie d'arrêt unidirectionnel dans un sens et une rampe dans l'autre et étant, dans leur état normal, dans l'alignement de la paroi extérieure cylindrique du fourreau 14. Elles sont, de cette façon, peu accessibles de l'extérieur, ce qui évite toute manipulation accidentelle par le personnel médical. Naturellement, il convient que les ergots 19 et 20 puissent être écartés provisoirement de l'extérieur par la machine de montage pour la mise en place de la seringue à l'état armé de la figure 1, afin de laisser passer l'épaulement 24, ainsi que tout autre épaulement éventuel de même sens, comme par exemple, l'épaulement 25 montré figure 1, situé sur la paroi extérieure du corps 1 le long de la course de rentrée du corps dans le fourreau au-dessous du niveau des ergots 20.

Dans le cas d'une utilisation prévue par des personnes tentées de réutiliser par tout moyen la seringue après une première injection, il est possible d'adjoindre en fin de montage une bague de protection 26, fixée par collage, soudage ou autrement sur le fourreau 14 pour recouvrir les ergots 19 et 20 et les rendre ainsi totalement inaccessibles, tout en leur permettant néanmoins de jouer librement leur rôle et de s'écarter au moment voulu : les ergots 19 lors du désarmement en fin de course du piston 13 dans la seringue, et les ergots 20 lors du recul du corps dans le fourreau après le désarmement.

Cette bague de protection 26 est de préférence étanche à l'air, de sorte qu'en munissant le fourreau d'un capuchon protecteur 27 (figure 3) également étanche, on peut stériliser la seringue montée sans emballage individuel ainsi que cela se pratique pour certaines seringues à insuline.

On notera que de manière avantageuse, il a été prévu sur le corps 1 un évidement 27 permettant lorsque la seringue est en position armée, aux ergots 20 d'être au repos comme les ergots 19 (i.e. ils ne sont soumis à aucune contrainte). On préserve ainsi l'intégrité et les qualités élastiques des deux ergots antagonistes pendant le stockage de la seringue montée.

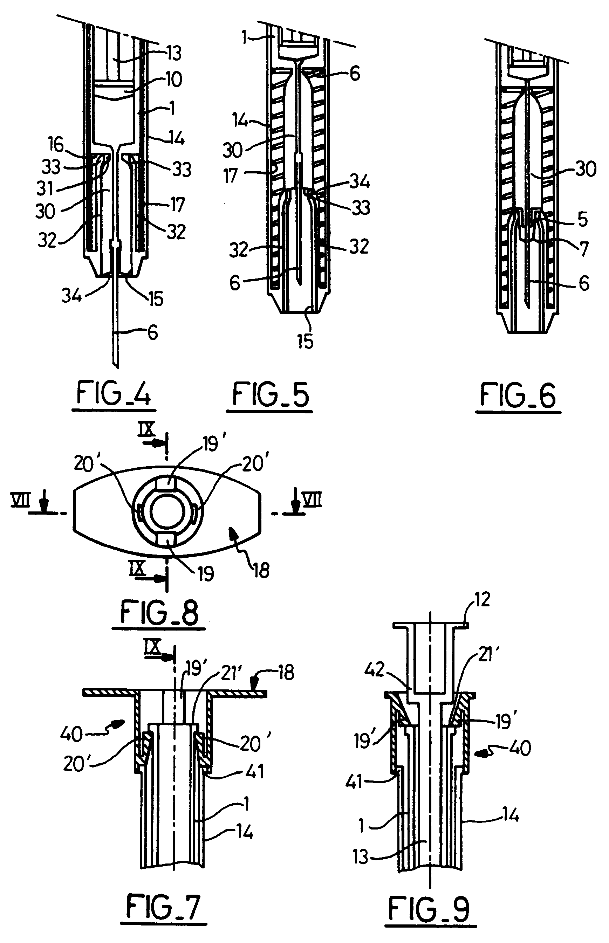

Le second mode de réalisation illustré sur les figures 4 à 6 diffère du premier essentiellement par la réalisation des seconds ergots de blocage anti-retour.

On retrouve dans ce mode de réalisation, le corps 1 et son piston intérieur 13 à étanchéité coulissante 10, ainsi qu'un fourreau coulissant extérieur 14 et un ressort 17 maintenu entre l'extrémité du fourreau 14 et un épaulement 16 à l'avant du corps 1.

Le corps se prolonge encore à l'avant par un mandrin alésé porte-aiguille 30 sensiblement cylindrique, d'une longueur égale à celle de l'aiguille 6 majorée de quelques millimètres, et présentant à sa base, du côté de la seringue, un rétrécissement 31.

Le fourreau 14 présente à ses extrémités six pattes flexibles 32 axiales réparties circonférentiellement autour de l'orifice 15 et munies à leurs extrémités rentrées d'un ergot 33 dirigé radialement vers l'intérieur.

Avant usage, le corps 1 de seringue est présenté enfoncé à fond dans le fourreau 14 (figure 4) : le mandrin porte-aiguille 30 vient alors affleurer le bord de l'orifice 15, tandis que les pattes 32 entourent ledit mandrin et sont au repos puisque leurs ergots saillants 33 se logent sans difficulté au niveau de la partie rétrécie 31. Le ressort 17 est bandé : la seringue est armée.

Le fonctionnement de la seringue est identique à celui du premier mode de réalisation en ce qui concerne le désarmement. Mais, lors du recul qui s'ensuit du corps 1 dans le fourreau 14, les pattes flexibles 32 s'écartent autour du mandrin 30 à cause des ergots 33 puis, grâce à leur élasticité, viennent en fin de course se repositionner (figure 5) de façon à bloquer le corps 1 de la seringue en butée par l'épaulement 34, formé par l'extrémité du mandrin 30, s'appuyant sur les ergots 33. L'épaulement 34 et les ergots 33 sont conformés de manière complémentaire concave-convexe pour que tout accroissement de la poussée renforce l'action de blocage. Au reste, si l'on force sur le poussoir, la partie amincie 31, plus fragile, peut se casser afin d' éliminer radicalement toute possibilité de réutilisation.

Alors que sur les figures 4 et 5, on a représenté une aiguille fixe collée, il est tout à fait possible, avec le même mode de réalisation, de terminer le mandrin porte-aiguille 30 par un téton 5 de fixation d'une aiguille interchangeable 6 portant elle-même son embout de fixation 7 (figure 6).

La machine de montage des seringues doit comporter un dispositif d'écartement provisoire des pattes 32 pour permettre le passage initial du mandrin 30.

Il est à noter que même dans le mode de réalisation des figures 1 à 3, il est possible de prévoir à la base de l'embout 4, 5 une partie rétrécie frangible, analogue à celle du mandrin 30. Dans ce cas, on profite de ce rétrécissement pour y fixer fermement la dernière spire du ressort 17. Ainsi, même si l'on parvient à découper le fourreau 14 pour récupérer la seringue et son aiguille, tout essai de dégagement du ressort 17 hors de la zone rétrécie de moindre résistance entraînera une cassure et donc une séparation de l'embout 4, 5 d'avec le corps de seringue. Toute réutilisation de l'aiguille et/ou de la seringue deviendra impossible.

Selon le mode de réalisation des figures 7 à 9, les ergots 19 et 20 sont remplacés par des ergots 19' et 20' portés par un manchon 40 sensiblement cylindrique rendu solidaire du fourreau 14 par tout moyen approprié tel que collage, soudage à ultrasons, thermosoudage, etc. d'un bord 41 du fourreau 14 dans une rainure du manchon 40. Le manchon 40 porte lui-même la collerette d'appui 18 de la seringue.

Les ergots 19' et 20' sont prévus à l'intérieur dudit manchon 40 et peuvent être réalisés d'une seule pièce avec celui-ci, par exemple par moulage. Les deux ergots 19' sont dirigés vers le base et sont susceptibles de venir coopérer avec l'épaulement 21' du corps 1 formé par son bord supérieur pour la position d'armement, ou avec un second épaulement non représenté situé plus bas sur le corps 1 pour la position désarmée. Le désarmement est déclenché par la coopération d'un renflement 42 prévu à l'arrière de la tige 13 de piston et destiné, d'une part, à écarter les ergots 19' en fin de course et, d'autre part, à s'appuyer sur le bord 21'.

Les ergots 20' sont placés en quadrature avec les ergots 19' et en sens inverse pour coopérer après le recul du corps de seringue avec un épaulement anti-retour approprié placé sur le corps 1.

Ce mode de réalisation présente de grands avantages de montage et de sécurité. Au montage en effet, aucun dispositif d'écartement temporaire des ergots n'est nécessaire, il suffit d'enfiler à fond le corps 1 dans le fourreau 14, de venir coiffer le tout par le manchon 40 qu'on fixe alors, puis de mettre le piston en place.

Le corps 1 adapté à ce mode de réalisation peut avoir une forme particulièrement simple par exemple une forme cylindrique portant à son extrémité proximale ou près de celle-ci une collerette ou filet formant le rebord 21' du premier épaulement, et plus loin sur son corps ou de son extrémité distale, un filet périphérique formant d'un côté le second épaulement et de l'autre le troisième épaulement.

## Revendications

1. Seringue à aiguille auto-escamotable, comprenant un corps (1) portant à une extrémité des moyens (5) de support d'aiguille d'injection (6), un piston (13) mobile dans ledit corps, un fourreau (14) protecteur à l'intérieur duquel est susceptible de coulisser ledit corps (1), un ressort (17) disposé entre le fourreau (14) et le corps (1), des moyens d'accrochage du fourreau (14) sur le corps (1) comprenant un premier et un second épaulement (21, 23) sur la paroi externe du corps (1) et au moins un premier ergot (19) souple porté par le fourreau (14) de manière à coopérer en butée avec le premier épaulement (21) pour définir une première position de blocage du corps (1) par rapport au fourreau (14) dans un premier sens, le ressort (17) étant comprimé et l'aiguille (6) sortie du fourreau dans cette première position, et avec le second épaulement (23) pour définir une seconde position de blocage dans le même sens, le ressort (17) étant au moins partiellement débandé et l'aiguille (6) étant rentrée dans le fourreau (14) dans cette seconde position, le passage de la première position à la seconde position se faisant par la coopération du piston (13) avec ledit premier ergot souple (19) pour libérer en fin de course dudit piston (13) l'ergot (19) du premier épaulement (21) et provoquer ainsi sous l'effet du ressort (17) le recul du corps (1) dans le fourreau (14) jusqu'à son blocage en seconde position, caractérisée par au moins un second ergot souple (20, 20', 33) et un troisième épaulement (24), de sens contraire respectivement au premier ergot (19) et aux deux premiers épaulements (21, 23), positionnés respectivement sur le fourreau (14) et la paroi du corps (1) pour bloquer le corps (1) dans le fourreau (14), lorsqu'il est dans la seconde position de blocage, dans le sens inverse au premier sens.

2. Seringue selon la revendication 1, caractérisée en ce que la partie inférieure (5) du corps (1) est susceptible de recevoir un embout porte-aiguille (7) et en ce que l'extrémité inférieure du fourreau comporte un orifice (15) de passage pour ledit embout (7).

3. Seringue selon l'une quelconque des revendications 1 ou 2, caractérisée en ce qu'au moins certains des ergots (20, 19, 33)sont souples et réalisés d'un seul tenant avec le fourreau (14).

4. Seringue selon l'une quelconque des revendications 1 à 3 caractérisée en ce qu'au moins certains des ergots (20, 19) sont protégés de l'extérieur par une paroi en forme de manchon (26, 40) solidaire du fourreau (14).

5. Seringue selon l'une quelconque des revendications 1 à 4, caractérisée en ce que le corps (1) comprend un mandrin (30) porte-aiguille et en ce qu'au moins certains desdits ergots (33) sont prévus à l'intérieur du fourreau (14) et autour dudit mandrin (3).

6. Seringue selon la revendication 5, caractérisée en ce que le mandrin (30) porte-aiguille comporte une zone amincie (31).

7. Seringue selon l'une quelconque des revendications 1 à 6 caractérisée en ce qu'une extrémité du ressort(17) est engagée dans une zone de moindre résistance ménagée sur le corps (1).

8. Seringue selon l'une quelconque des revendications 1 à 7 caractérisée en ce que le piston (13) de seringue comporte une partie (22, 42) susceptible de venir directement en contact avec les premiers ergots (19).

## Claims

1. A syringe with a self-retracting needle, comprising a body (1) carrying at one end a means (5) for supporting an injection needle (6), a plunger (13) which is mobable within said body, a protective sheath (14) within which said body (1) can slide, a spring (17) disposed between said sheath (14) and said body (1), a means for catching said sheath (14) on the body (1) comprising first and second shoulders (21, 23) on the outer wall of said body (1) and at least a first flexible toe (19) borne by said sheath (14) so as to cooperate in abutment with said first shoulder (21) for defining a frist blocking position of said body (1) in relation to said sheath (14) in a first direction, said spring (17) being compressed and said needle (6) protruding from said sheath in this first position, and with said second shoulder (23) for defining a second blocking position in the same direction, said spring (17) being at least partially released and said needle (6) being withdrawn inside said sheath (14) in this second position, the transition from said first position to said second position being made by cooperation of said plunger (13) with said first flexible toe (19) for freeing said toe (19) of said first shoulder (21) at the end of stroke of said plunger (13), thereby causing said body (1) to retract into said sheath (14) under the effect of said spring (17) until it blocks in said second position, characterized in that it comprises at least a second flexible toe (20, 20', 33) and a third shoulder (24), respectively in the opposite direction to said first flexible toe (19) and two first shoulders (21, 23), respectively positioned on said sheath (14) and wall of said body (1) for blocking said body (1) in said sheath (14) when it is in said second blocking position, in the opposite direction to said first direction.

2. The syringe as claimed in claim 1, characterized in that the lower part (5) of said body (1) is arranged for receiving a needle-holding connection end (7) and the bottom end of said sheath comprises a hole (15) for the passage of said connection end.

3. The syringe as claimed in either claim 1 or 2, characterized in that at least certain toes (20, 19, 33) are flexible and made integral with said sheath (14).

4. The syringe as claimed in any one of claims 1 to 3, characterized in that at least certain toes (20, 19) are protected on the outside by a wall in the form of a sleeve (26, 40) fixed with said sheath.

5. The syringe as claimed in any one of claims 1 to 4, characterized in that said body (1) comprises a needle-holding chuck (30) and at least certain of said toes (33) are provided inside said sheath (14) and around said chuck.

6. The syringe as claimed in any one of claims 1 to 5, characteried in that said needle-holding chuck (30) comprises a restricted portion (31).

7. The syringe as claimed in any one of claims 1 to 6, characterized in that an end of said spring (17) is lodged in an area of lesser resistance provided on said body (1).

8. The syringe as claimed in any one of claims 1 to 7, characterized in that said syringe plunger (13) comprises a part (22, 42) arranged for directly engaging with said first toes (19).

## Patentansprüche

1. Spritze mit automatisch sich zurückziehender Nadel, mit einem Körper (1), der an einem Ende Mittel (5) zum Halten der Injektionsnadel (6) trägt, einem Kolben (13), der im Körper beweglich ist, einer Schutzhülse (14), in welche der Körper (1) gleiten kann, einer Feder (17), die zwischen der Hülse (14) und dem Körper (1) angeordnet ist, und Mitteln zum Einhaken der Hülse (14) am Körper (1), die eine erste und eine zweite Schulter (21, 23) an der Außenwand des Körpers (1) sowie wenigstens einen ersten elastischen Vorsprung (19) umfassen, den die Hülse (14) trägt, derart, daß der erste elastische Vorsprung (19) mit der ersten Schulter (21) durch Anschlag zusammenwirkt, um eine erste Blockierstellung des Körpers (1) in bezug auf die Hülse (14) in einer ersten Richtung zu definieren, wobei in dieser ersten Stellung die Feder (17) zusammengedrückt ist und die Nadel (6) aus der Hülse austritt, und mit der zweiten Schulter (23) zusammenwirkt, um eine zweite Blockierstellung in derselben Richtung zu definieren, wobei in dieser zweiten Stellung die Feder (17) wenigstens teilweise entspannt ist und die Nadel (6) in die Hülse (14) zurückgezogen ist, wobei der Übergang von der ersten Stellung in die zweite Stellung durch Zusammenwirken des Kolbens (13) mit dem ersten elastischen Vorsprung (19) geschieht, wodurch am Ende des Hubes des Kolbens (13) der Vorsprung (19) von der ersten Schulter (21) gelöst wird und somit durch die Wirkung der Feder (17) das Zurückziehen des Körpers (1) in die Hülse (14) bis in seine zweite Blockierstellung bewirkt wird, gekennzeichnet durch wenigstens einen zweiten elastischen Vorsprung (20, 20', 33) und eine dritte Schulter (24) mit einem dem ersten Vorsprung (19) bzw. den beiden ersten Schultern (21, 23) entgegengesetzten Richtungsinn, die an der Hülse (14) bzw. an der Wand des Körpers (1) angeordnet sind, um den Körper (1) in der Hülse (14) in der zur ersten Richtung entgegengesetzten Richtung zu blockieren, wenn er sich in der zweiten Blockierstellung befindet.

2. Spritze gemäß Anspruch 1, dadurch gekennzeichnet, daß der untere Teil (5) des Körpers ein Nadelträger-Ansatzstück (7) aufnehmen kann und daß das untere Ende der Hülse eine Durchlaßöffnung (15) für das Ansatzstück (7) enthält.

3. Spritze gemäß einem der Ansprüche 1 oder 2, dadurch gekennzeichnet, daß wenigstens bestimmte der Vorsprünge (20, 19, 33) elastisch sind und in einem Stück mit der Hülse (14) verwirklicht sind.

4. Spritze gemäß einem der Ansprüche 1 bis 3, dadurch gekennzeichnet, daß wenigstens bestimmte der Vorsprünge (20, 19) nach außen durch eine Wand in Form einer Muffe (26, 40), die mit der Hülse (14) verbunden ist, geschützt sind.

5. Spritze gemäß einem der Ansprüche 1 bis 4, dadurch gekennzeichnet, daß der Körper (1) einen Nadelträger-Dorn (30) aufweist und daß wenigstens bestimmte der Vorsprünge (33) innerhalb der Hülse (14) und um den Dorn (3) vorgesehen sind.

6. Spritze gemäß Anspruch 5, dadurch gekennzeichnet, daß der Nadelträger-Dorn (30) eine spitz zulaufende Zone (31) enthält.

7. Spritze gemäß einem der Ansprüche 1 bis 6, dadurch gekennzeichnet, daß ein Ende der Feder (17) mit einer Zone geringeren Widerstandes in Eingriff ist, die im Körper (1) ausgebildet ist.

8. Spritze gemäß einem der Ansprüche 1 bis 7, dadurch gekennzeichnet, daß der Kolben (13) der Spritze einen Teil (22, 42) aufweist, der direkt mit den ersten Vorsprüngen (19) in Kontakt gelangen kann.
